(19) Europäisches Patentamt
European Patent Office
Office européen des brevets

(11) Veröffentlichungsnummer: 0 365 483 B1

(12) # EUROPÄISCHE PATENTSCHRIFT

(45) Veröffentlichungstag der Patentschrift: **29.12.93**

(51) Int. Cl.[5]: **C08K 5/00**, C08L 57/06, C07C 323/62, //(C08K5/00,5:37, 5:09),(C08K5/00,5:37,5:13)

(21) Anmeldenummer: **89810777.6**

(22) Anmeldetag: **11.10.89**

Die Akte enthält technische Angaben, die nach dem Eingang der Anmeldung eingereicht wurden und die nicht in dieser Patentschrift enthalten sind.

(54) **Mercaptobenzoesäureester als Stabilisatoren für chlorhaltige Polymerisate.**

(30) Priorität: **20.10.88 CH 3912/88**

(43) Veröffentlichungstag der Anmeldung:
**25.04.90 Patentblatt 90/17**

(45) Bekanntmachung des Hinweises auf die Patenterteilung:
**29.12.93 Patentblatt 93/52**

(84) Benannte Vertragsstaaten:
**BE DE FR GB IT**

(56) Entgegenhaltungen:
**EP-A- 0 001 631**
**EP-A- 0 133 130**
**EP-A- 0 292 672**
**DE-A- 1 217 609**

(73) Patentinhaber: **CIBA-GEIGY AG**
**Klybeckstrasse 141**
**CH-4002 Basel(CH)**

(72) Erfinder: **Wehner, Wolfgang, Dr.**
**Wetzbach 34**
**D-6144 Zwingenberg(DE)**
Erfinder: **Abeler, Gerd, Dr.**
**Alter Wixhäuser Weg 61**
**D-6100 Darmstadt(DE)**

EP 0 365 483 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von Mercaptobenzoesäureestern zum Stabilisieren von chlorhaltigen Polymerisaten gegen thermischen Abbau sowie die stabilisierten chlorhaltigen Polymerisate und neue Mercaptobenzoesäureester.

Es ist bekannt, dass chlorhaltige Polymerisate gegen den schädigenden Einfluss von Licht und Wärme, insbesondere bei der Verarbeitung zu Formteilen, geschützt werden müssen. Einige Mercaptobenzoesäureester und deren Verwendung als Stabilisatoren werden beispielsweise in US-A-3 242 133, US-A-4 361 665, EP-A-39 159, DE-A-1 217 609 und GB-A-936 770 beschrieben. Ein Verfahren zum Vernetzen von halogenierten Polymeren mit Hilfe von Alkali- und Erdalkalithiolaten ist aus US-A-4 647 629 bekannt. EP-A-321 405 beschreibt die Verwendung von Mercaptobenzoesaureestern bei der Herstellung von Organozinnalkoxy-carbonylphenylmercaptiden. Die Verwendung von Mercaptoacetaten und Mercaptopropionaten auf festen Trägermaterialien zum Stabilisieren von chlorhaltigen thermoplastischen Kunststoffen wird in EP-A-1 631 beschrieben.

Die vorliegende Erfindung betrifft Zusammensetzungen enthaltend a) ein chlorhaltiges Polymerisat, b) mindestens ein Me(II)-Carboxylat und/oder Me(II)-Phenolat, wobei Me(II) Ba, Ca, Mg, Cd, Sr oder Zn bedeutet und c) mindestens eine Verbindung der Formel I,

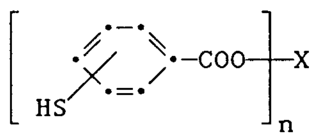

(I)

worin n 1, 2, 3, 4 oder 6 bedeutet und wenn n 1 ist, X $C_1$-$C_{20}$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, $C_7$-$C_9$-Phenylalkyl, durch 1 bis 3 Reste substituiertes Phenyl oder am Phenylring durch 1 bis 3 Reste substituiertes $C_7$-$C_9$-Phenylalkyl bedeutet, wobei die Reste aus der Gruppe bestehend aus $C_1$-$C_4$-Alkyl, Chlor, Hydroxy, Methoxy und Ethoxy ausgewählt sind; wenn n 2 ist, X $C_2$-$C_{12}$-Alkylen, durch Phenyl substituiertes $C_2$-$C_{12}$-Alkylen oder durch Sauerstoff- oder Schwefelatome unterbrochenes $C_2$-$C_{12}$-Alkylen bedeutet; wenn n 3 ist, X die Gruppe der Formel II

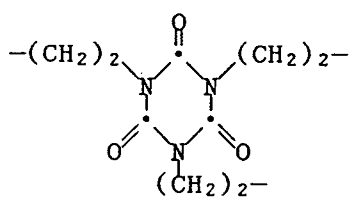

(II)

oder $C_3$-$C_7$-Alkantriyl darstellt; wenn n 4 ist, X $C_4$-$C_{10}$-Alkantetrayl oder die Gruppe der Formel IIIa oder IIIb ist

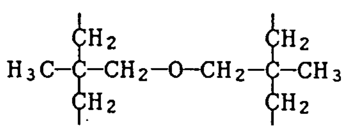

(IIIa)

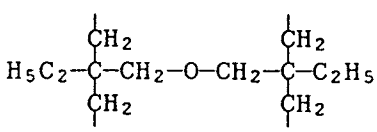

(IIIb)

und wenn n 6 bedeutet, X die Gruppe der Formel IV

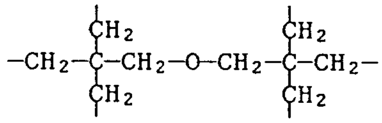

(IV)

darstellt,
mit den Bedingungen, dass

(1) Thiosalicylsäure-3-(3-t-butyl-4-hydroxyphenyl)-propylester als Komponente c) ausgeschlossen ist und

(2) die Zusammensetzungen kein olefinisch ungesättigtes Terpen oder Sauerstoff-, Kohlenwasserstoff- oder Halogen-Derivat eines olefinisch ungesättigten Terpens enthalten.

Die SH-Gruppe in den Verbindungen der Formel I kann sich in ortho-, meta- oder para-Stellung befinden.

$C_1$-$C_{20}$-Alkyl kann geradkettig oder verzweigt sein und bedeutet beispielsweise Methyl, Ethyl, Propyl, n-Butyl, i-Butyl, t-Butyl, Pentyl, Hexyl, Heptyl, n-Octyl, i-Octyl, i-Nonyl, n-Decyl, n-Dodecyl oder n-Octadecyl. X ist bevorzugt $C_1$-$C_{12}$-Alkyl, insbesondere $C_6$-$C_{12}$-Alkyl, z.B. 2-Ethylhexyl.

$C_3$-$C_{20}$-Alkenyl kann geradkettig oder verzweigt sein und bedeutet zum Beispiel Allyl, 2-Methallyl, 3-Methylbut-2-enyl, 3-Methylbut-3-enyl, Hexenyl, Decenyl, Undecenyl, Heptadecenyl oder Oleyl. Bevorzugte Bedeutungen für X als Alkenyl sind Allyl, Methallyl und Oleyl.

X bedeutet als $C_5$-$C_{12}$-Cycloalkyl, welches gegebenenfalls durch $C_1$-$C_4$-Alkyl, insbesondere Methyl, substituiert sein kann, zum Beispiel Cyclopentyl, Cyclohexyl, Methylcyclohexyl, 4-Butylcyclohexyl, Cycloheptyl, Cyclooctyl oder Cyclododecyl. $C_5$-$C_7$-Cycloalkyl, insbesondere Cyclohexyl, ist bevorzugt.

$C_7$-$C_9$-Phenylalkyl bedeutet bevorzugt Benzyl oder Phenylethyl.

Phenyl, welches durch 1 bis 3 Reste substituiert ist, bedeutet beispielsweise o-, m- oder p-Chlorphenyl, 2,3-Dichlorphenyl, 2,4-Dichlorphenyl, 2,5-Dichlorphenyl, 2,6-Dichlorphenyl, 3,4-Dichlorphenyl, 2,4,5-Trichlorphenyl, 2,4,6-Trichlorphenyl, o-, m- oder p-Hydroxyphenyl, o-, m-oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-4-tert-butylphenyl, 2-Ethylphenyl, 2,6-Diethylphenyl, 2,6-Diethyl-4-methylphenyl, 2,6-Diisopropylphenyl, 4-tert-Butylphenyl, 3,5-Di-tert-butylphenyl, 2-Chlor-6-methylphenyl, 3-Chlor-2-methylphenyl, 3-Chlor-4-methylphenyl, 4-Chlor-2-methylphenyl, 5-Chlor-2-methylphenyl, 2,6-Dichlor-3-methylphenyl, 2-Hydroxy-4-methylphenyl, 3-Hydroxy-4-methylphenyl, o-, m- oder p- Methoxyphenyl, o-, m- oder p-Ethoxyphenyl, 2,4-Dimethoxyphenyl, 2,5-Dimethoxyphenyl, 2,5-Diethoxyphenyl, 2-Methoxy-5-methylphenyl, 4-Methoxy-2-methylphenyl, 3-Chlor-4-methoxyphenyl, 3-Chlor-6-methoxyphenyl, 3-Chlor-4,6-dimethoxyphenyl oder 4-Chlor-2,5-dimethoxyphenyl.

$C_7$-$C_9$-Phenylalkyl, welches durch 1 bis 3 Reste substituiert ist, bedeutet zum Beispiel o-, m- oder p-Chlorbenzyl, 2,3-Dichlorbenzyl, 2,4-Dichlorbenzyl, 2,5-Dichlorbenzyl, 2,6-Dichlorbenzyl, 3,4-Dichlorbenzyl, 2,4,5-Trichlorbenzyl, 2,4,6-Trichlorbenzyl, o-, m- oder p-Hydroxybenzyl, o-, m- oder p-Methylbenzyl, 2,3-Dimethylbenzyl, 2,4-Dimethylbenzyl, 2,5-Dimethylbenzyl, 2,6-Dimethylbenzyl, 3,4-Dimethylbenzyl, 3,5-Dimethylbenzyl, 2-Methyl-4-tert-butylbenzyl, 2-Ethylbenzyl, 2,6-Diethylbenzyl, 2,6-Diethyl-4-methylbenzyl, 2,6-Diisopropylbenzyl, 4-tert-Butylbenzyl, 2-Chlor-6-methylbenzyl, 3-Chlor-2-methylbenzyl, 3-Chlor-4-methylbenzyl, 4-Chlor-2-methylbenzyl, 5-Chlor-2-methylbenzyl, 2,6-Dichlor-3-methylbenzyl, 2-Hydroxy-4-methylbenzyl, 3-Hydroxy-4-methylbenzyl, o-, m- oder p- Methoxybenzyl, o-, m- oder p-Ethoxybenzyl, 2,4-Dimethoxybenzyl, 2,5-Dimethoxybenzyl, 2,5-Diethoxybenzyl, 2-Methoxy-5-methylbenzyl, 4-Methoxy-2-methylbenzyl, 3-Chlor-4-methoxybenzyl, 3-Chlor-6-methoxybenzyl, 3-Chlor-4,6-dimethoxybenzyl oder 4-Chlor-2,5-dimethoxybenzyl.

X bedeutet als $C_2$-$C_{12}$-Alkylen zum Beispiel Dimethylen, Trimethylen, Tetramethylen, Hexamethylen, 2,2-Dimethyltrimethylen, 2-Ethyl-2-butyltrimethylen, 2-Methyl-2-propyltrimethylen, Octamethylen, Nonamethylen, Decamethylen oder Dodecamethylen. Bevorzugt ist $C_2$-$C_6$-Alkylen.

X bedeutet als $C_2$-$C_{12}$-Alkylen, welches durch Phenyl substituiert ist, bevorzugt 2-Methyl-2-phenyltrimethylen.

X bedeutet als $C_2$-$C_{12}$-Alkylen, welches durch, bevorzugt bis zu drei, Sauerstoff- oder Schwefelatome unterbrochen ist, zum Beispiel 3-Thiapentamethylen-1,5, 3,6-Dithiaoctamethylen-1,8, 3-Oxapentamethylen-1,5, 4-Oxaheptamethylen-1,7 oder 3,6-Dioxaoctamethylen-1,8. Zwischen den Heteroatomen befindet sich bevorzugt eine Dimethylen-, Trimethylen- oder Tetramethylengruppe.

X bedeutet als $C_3$-$C_7$-Alkantriyl insbesondere

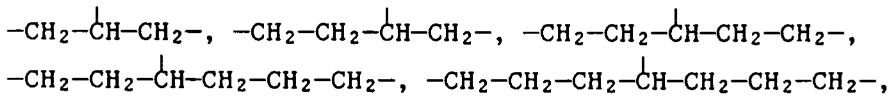

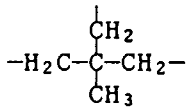 (Va) oder 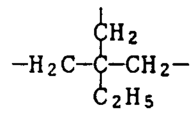 (Vb).

Die Gruppen Va und Vb sind bevorzugt.

X bedeutet als $C_4$-$C_{10}$-Alkantetrayl insbesondere

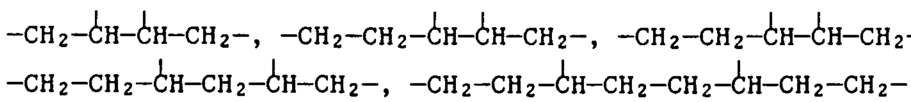 oder

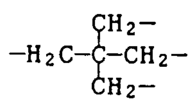 (Vc).

Die Gruppe Vc ist bevorzugt.

Bevorzugt sind Zusammensetzungen enthaltend als Komponente c) eine Verbindung der Formel I, worin n 1, 2, 3 oder 4 bedeutet und wenn n 1 ist, X $C_1$-$C_{12}$-Alkyl, $C_3$-$C_{18}$-Alkenyl, $C_5$-$C_7$-Cycloalkyl, durch Methyl substituiertes Cyclohexyl, Phenyl oder $C_7$-$C_9$-Phenylalkyl bedeutet; wenn n 2 ist, X $C_2$-$C_6$-Alkylen darstellt; wenn n 3 ist, X die Gruppe der Formel II oder die Gruppe der Formel Va oder Vb

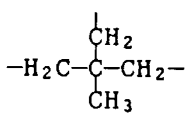 (Va) , 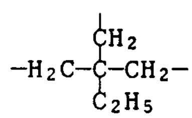 (Vb)

bedeutet und wenn n 4 ist, X die Gruppe der Formel Vc

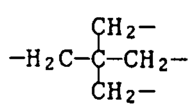 (Vc)

ist.

Ebenfalls bevorzugt sind Zusammensetzungen enthaltend als Komponente c) eine Verbindung der Formel I, worin n 1, 2, 3 oder 4 bedeutet und wenn n 1 ist, X $C_1$-$C_{12}$-Alkyl, Allyl, Methallyl, Oleyl, Cyclohexyl, Phenyl oder Benzyl bedeutet; wenn n 2 ist, X $C_2$-$C_6$-Alkylen darstellt; wenn n 3 ist, X die Gruppe der Formel Va oder Vb darstellt und wenn n 4 ist, X die Gruppe der Formel Vc bedeutet.

In den Verbindungen der Formel I bedeutet n bevorzugt 1 und X bevorzugt $C_1$-$C_{12}$-Alkyl.

Als Komponente c) wird die Verbindung 2-Ethylhexyl-thiosalicylat oder 1,6-Hexamethylen-bis-[thiosalicylat] besonders bevorzugt.

Die Komponente b) ist bevorzugt ein Me(II)-Carboxylat, wobei Me(II) Ba, Ca, Mg, Cd oder Zn bedeutet. Bei den Carboxylaten handelt es sich bevorzugt um Salze von Carbonsäuren mit 7 bis 20 C-Atomen, z.B. Benzoate, Alkanoate oder Alkenoate, bevorzugt Stearate, Oleate, Laurate, Palmitate, Hydroxystearate oder 2-Ethylhexanoate. Besonders bevorzugt sind Stearate, Oleate oder p-tert-Butylbenzoate.

Gemische aus Ba/Zn- oder Ca/Zn-Carboxylaten werden als Komponente b) ebenfalls besonders bevorzugt.

Bedeutet die Komponente b) ein Me(II)-Phenolat, so handelt es sich insbesondere um $C_7$-$C_{20}$-Alkylphenolate, beispielsweise Nonylphenolat.

Gemäss einer weiteren Bevorzugung enthalten die erfindungsgemässen Zusammensetzungen als zusätzliche Komponente d) eine Epoxyverbindung und/oder ein Phosphit.

Bei der Epoxyverbindung handelt es sich bevorzugt um epoxidierte Oele und epoxidierte Fettsäureester, z.B. epoxidiertes Sojabohnenöl, epoxidiertes Butyloleat und epoxidiertes Octyloleat.

Bei den Phosphiten handelt es sich bevorzugt um solche der Formeln

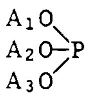, 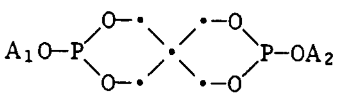, 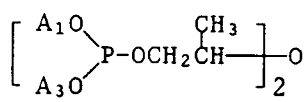, 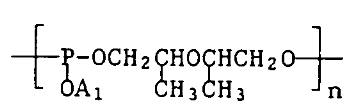,

worin $A_1$, $A_2$ und $A_3$ unabhängig voneinander $C_4$-$C_{18}$-Alkyl, $C_6$-$C_{18}$-Alkenyl, $C_5$-$C_7$-Cycloalkyl, Phenyl oder durch ein bis drei $C_1$-$C_{12}$-Alkylgruppen substituiertes Phenyl bedeuten.

Beispiele sind Trioctyl-, Tridecyl-, Tridodecyl-, Tritetradecyl-, Tristearyl-, Trioleyl-, Triphenyl-, Trikresyl-, Tris-p-nonylphenyl- und Tricyclohexylphosphit. Bevorzugt sind die Aryldialkyl- sowie die Alkyldiarylphosphite, wie z.B. Phenyldidecyl-, (2,4-Di-tert-butylphenyl)didodecyl-, (2,6-Di-tert-butylphenyl)didodecylphosphit und die Dialkyl- und Diaryl-pentaerythrit-diphosphite, wie z.B. Distearylpentaerythrit- diphosphit. Ebenfalls bevorzugt sind die Tetraphenyl- und Tetraalkyl-[dipropylenglykol-1,2]-diphosphite und die Poly-[dipropylenglykol-1,2-phenylphosphite] sowie die Poly-[dipropylenglykol-1,2-alkylphosphite].

Besonders bevorzugte organische Phosphite sind Distearyl-pentaerythritdiphosphit, Tris(nonylphenyl)-phosphit, Phenyldidecylphosphit, Tetraphenyl-[dipropylenglykol-1,2]-diphosphit und Poly-[dipropylenglykol-1,2-phenylphosphit].

Die Me(II)-Carboxylate oder -Phenolate können in dem zu stabilisierenden Material in einer dem Fachmann bekannten Konzentration vorliegen, wie zum Beispiel in Mengen von 0,05 bis 5 Gew.%.

Die Phosphite werden z.B. in Konzentrationen von 0,3 bis 5, vorzugsweise 0,5 bis 1 Gew.% und die Epoxyverbindungen, wie z.B. das epoxidierte Sojabohnenöl, zweckmässig in Konzentrationen von 1 bis 8, vorzugsweise 1 bis 3 Gew.% eingesetzt.

Die Verbindungen der Formel I werden beispielsweise in Mengen von 0,05 bis 5, bevorzugt 0,05 bis 1, insbesondere 0,1 bis 0,7 Gew.% in das chlorhaltige Polymerisat eingearbeitet.

Die Angabe Gew.% bezieht sich jeweils auf das zu stabilisierende Material.

Bei den chlorhaltigen Polymerisaten handelt es sich bevorzugt um Vinylchloridhomopolymere oder -copolymere. Als Comonomere für die Copolymerisate kommen z.B. in Frage: Vinylacetat, Vinylidenchlorid, Transdichlorethen, Ethylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA, ABS und MBS. Bevorzugte Substrate sind auch Mischungen der vorstehend genannten Homo- und Copolymerisate, insbesondere Vinylchlorid-Homopolymerisate, mit anderen thermoplastischen oder/und elastomeren Polymeren, insbesondere mit ABS, MBS, NBR, SAN, EVA.

Weiterhin bevorzugt sind Suspensions- und Massepolymere sowie Emulsionspolymere.

Als chlorhaltiges Polymerisat ist Polyvinylchlorid besonders bevorzugt.

Je nach dem Verwendungszweck der Polymerisate können vor oder bei der Einarbeitung der Stabilisatoren auch weitere Zusätze eingearbeitet werden, wie zum Beispiel phenolische Antioxidantien, Gleitmittel (bevorzugt Montanwachse oder Glycerinester, Fettsäureester, Paraffine, Amidwachse, Stearinsäure, Mono- und Dihydroxystearinsäure, höhere Fettalkohole), Weichmacher, Füllstoffe, Russ, Asbest, Kaolin, Talk, Glasfasern, Modifikatoren (wie etwa Schlagzäh-Zusätze), Verarbeitungshilfen (z.B. Polymethacrylsäureester), optische Aufheller, Pigmente, Lichtschutzmittel, UV-Absorber, Flammschutzmittel oder Antistatika.

Weitere mögliche Zusätze sind ferner β-Aminocrotonate, z.B. die in DE-A-804 442, DE-A-807 207 und JP-A-75/17454 beschriebenen Verbindungen, Pyrrole, z.B. die in EP-A-22 087 angegebenen Verbindungen, Aminouracile, z.B. die in EP-A-65 934 offenbarten Verbindungen, Aminothiouracile, z.B. die aus EP-A-41 479 bekannten Verbindungen, Polyole, z.B. die in DE-A-3 019 910 beschriebenen Verbindungen, β-Diketone, z.B. die in DE-A-2 600 516 angegebenen Verbindungen, oder auch Gemische aus β-Diketonen und Hydrotalciten, wie z.B. in EP-A-63 180 beschrieben.

Die Einarbeitung der Stabilisatorkomponenten in das chlorhaltige Polymerisat erfolgt am günstigsten, wie üblich, auf einem Mischwalzwerk, z.B. einem 2-Walzenstuhl bei Temperaturen zwischen 150° und 200°C. Im allgemeinen lässt sich eine genügende Homogenisierung innerhalb von 5 bis 15 Minuten

erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachern gearbeitet.

Die Verbindungen der Formel I können in Analogie zu bekannten Verfahren hergestellt werden, beispielsweise durch Veresterung der entsprechenden Mercaptobenzoesäure oder wenn X $C_4$-$C_{20}$-Alkyl bedeutet, durch Umesterung eines Mercaptobenzoesäuremethylesters bzw. Mercaptobenzoesäureethylesters.

Ein weiterer Gegenstand der Erfindung sind die neuen Verbindungen der Formel IA,

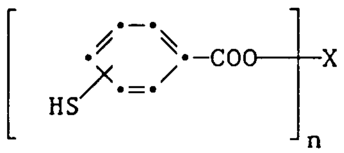
(IA)

worin n 2, 3, 4 oder 6 bedeutet und wenn n 2 ist, X durch Phenyl substituiertes $C_2$-$C_{12}$-Alkylen oder durch Schwefelatome unterbrochenes $C_2$-$C_{12}$-Alkylen bedeutet; wenn n 3 ist, X die Gruppe der Formel II

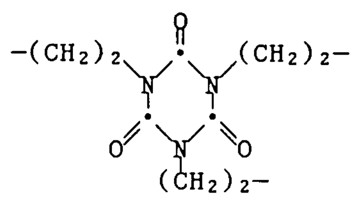
(II)

oder $C_3$-$C_7$-Alkantriyl darstellt; wenn n 4 ist, X $C_4$-$C_{10}$-Alkantetrayl oder die Gruppe der Formel IIIa oder IIIb ist

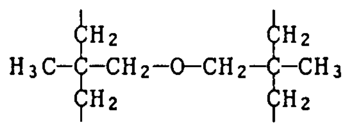
(IIIa)

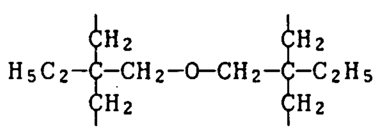
(IIIb)

und wenn n 6 bedeutet, X die Gruppe der Formel IV

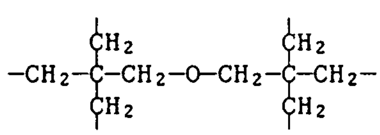
(IV)

darstellt.

Bevorzugt sind Verbindungen der Formel IA, worin n 3, 4 oder 6 bedeutet.

Die folgenden Beispiele erläutern die Erfindung weiter. Teile- und Prozentangaben beziehen sich darin, soweit nichts anderes angegeben ist, auf das Gewicht.

Beispiel 1: Herstellung von 2-Ethylhexyl-thiosalicylat.

77,1 g (0,5 Mol) Thiosalicylsäure werden unter Rühren in 250 ml heissem Isooctanol gelöst. Nach Zugabe von 100 ml Toluol und einer katalytischen Menge Paratoluolsulfonsäure wird das Reaktionsgemisch am Rückfluss erhitzt. Unter azeotropen Bedingungen scheiden sich nach 25 Stunden 8,5 ml Wasser (berechnet: 9 ml) ab. Der Rückstand wird mit einer Bicarbonatlösung ausgeschüttelt, anschliessend mit Wasser

gewaschen und getrocknet. Die flüchtigen Bestandteile werden im Vakuum entfernt. Man erhält das Produkt nach Rektifikation mit einer Quecksilberdiffusionspumpe.

Ausbeute:

109,7 g (= 82,4 % der Theorie)

Kochpunkt:

122-125 °C bei 0,07 mbar

Elementaranalyse:

| | |
|---|---|
| Berechnet: | SH, 12,4 % |
| Gefunden: | SH, 12,1 % |

Beispiele 2-6: Die in Tabelle 1 angegebenen Verbindungen werden in Analogie zu dem in Beispiel 1 beschriebenen Verfahren hergestellt.

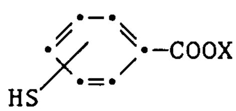

Tabelle 1:

| Bsp. | Stellung der SH-Gruppe | X | Brechungsindex $n_D^{20}$ | Siedepunkt °C | Elementaranalyse % S berechnet | % S gefunden |
|---|---|---|---|---|---|---|
| 2 | ortho | $-C_2H_5$ | 1,5738 | 97-98/ 0,8 mbar | 17,6 | 17,6 |
| 3 | ortho | $-C_4H_9$-i | 1,5538 | 123/ 14,7 mbar | 15,3 | 15,2 |
| 4 | meta | $-C_2H_5$ | 1,5617 | 133/ 7,33 mbar | 17,6 | 17,6 |
| 5 | para | $-C_2H_5$ | 1,5737 | 126/ 4,0 mbar | 17,6 | 17,6 |
| 6 | para | $-CH_2CH(C_2H_5)(CH_2)_3CH_3$ | 1,5232 | - | 12,0 | 11,8 |

Beispiel 7: Herstellung von Phenyl-thiosalicylat.

In einem 250-ml-Dreihalskolben wird unter Rühren ein Gemisch aus 69,4 g (0,45 Mol) Thiosalicylsäure und 42,3 g (0,45 Mol) Phenol bei 135 °C geschmolzen. Anschliessend werden bei 125-130 °C 24,5 g (0,16 Mol) Phosphoroxychlorid zugetropft, wobei HCl frei wird. Nach Beendigung der Zugabe (ca. 15 Min.) wird 30 Min. nachgerührt. Die erhaltene dunkelbraune, klare Lösung wird in 500 ml Wasser eingerührt, wobei sich ein braunes Oel abscheidet. Dieses wird in Essigester aufgenommen. Die organische Phase wird dreimal

mit Sodalösung ausgeschüttelt, mit Wasser gewaschen, getrocknet und bis zum Rückstand eingeengt. Der noch phenolhaltige Rückstand wird destillativ gereinigt. Das Rohprodukt wird aus 250 ml abs. Methanol in Gegenwart von Aktivkohle umkristallisiert. Das Produkt liegt in Form von farblosen Kristallen vor, die einen Schmelzpunkt von 89°C besitzen. Die Ausbeute beträgt 36,0 g (= 35 % der Theorie).

Beispiel 8: Herstellung von 1,6-Hexamethylen-bis[thiosalicylat].

In einem 500-ml-Dreihalskolben wird ein Gemisch aus 69,4 g (0,45 Mol) Thiosalicylsäure, 17,7 g (0,15 Mol) Hexandiol-1,6 und einer Spatelspitze Paratoluolsulfonsäure unter Rühren in 150 ml Xylol am Rückfluss erhitzt.

Während der Umsetzung wird wiederholt Paratoluolsulfonsäure zugegeben. Innerhalb von 10 h scheiden sich 5,8 ml Wasser (berechnet: 5,4 ml) ab. Das noch heisse Reaktionsgemisch wird über einer Filterhilfe geklärt und das Filtrat wird bis zum Rückstand eingeengt. Der Rückstand wird in Essigester aufgenommen, dreimal mit Bicarbonat-Lösung ausgeschüttelt, mit $H_2O$ gewaschen und getrocknet. Die organische Phase wird erneut bis zum Rückstand eingeengt. Der Rückstand wird in 350 ml Aceton in Gegenwart von Aktivkohle gelöst. Aus dem Filtrat lässt sich mit Eiswasser unter Rühren ein hellgelber Niederschlag ausfällen, der aus Bis- und Monoester besteht. Der reine Bisester wird nach Methanolextraktion als nahezu farblose Substanz erhalten. Das Produkt besitzt einen Schmelzpunkt von 95°C.

Beispiel 9: Herstellung von Tris[thiosalicyloyloxyethyl]isocyanurat.

Die Herstellung erfolgt in Analogie zu Beispiel 8. Es werden 0,45 Mol Thiosalicylsäure und 0,1 Mol Tris-[hydroxyethyl]isocyanurat eingesetzt. Nach der Aufarbeitung erhält man 40,9 g eines hochviskosen Harzes, das aus einem Gemisch aus Tris- und Bisester besteht. Der reine Trisester wird nach Methanolextraktion als nahezu farblose Substanz erhalten. Das Produkt besitzt einen Schmelzpunkt von 91°C.

Beispiel 10: Eine Trockenmischung bestehend aus 100 Teilen S-PVC (®Solvic 264 GA), 3 Teilen epoxidiertem Sojabohnenöl, 0,35 Teilen Ca-Stearat, 0,15 Teilen Zn-Stearat, 0,55 Teilen Didecyl-phenylphosphit und 0,67 Teilen 2-Ethylhexylthiosalicylat (Beispiel 1) wird auf einem Mischwalzwerk 5 Minuten bei 180°C gewalzt. Vom gebildeten 0,3 mm dicken Walzfell werden Folienmuster in einem Trockenschrank bei 180°C thermisch belastet. Der Yellowness Index (YI) der Proben wird in regelmässigen Zeitabständen nach ASTM D 1925 bestimmt. Die Ergebnisse sind in Tabelle 2 aufgeführt.

Tabelle 2

| YI nach Belastungszeit in Minuten | | | | | | | |
|---|---|---|---|---|---|---|---|
| 0 | 5 | 10 | 15 | 20 | 25 | 30 | 35 |
| 2,4 | 3,7 | 4,2 | 5,2 | 7,7 | 8,9 | 14,6 | 23,7 |

Beispiel 11: Eine Trockenmischung bestehend aus 100 Teilen S-PVC (®Solvic 268GA), 3 Teilen epoxidiertem Sojabohnenöl, 0,35 Teilen Ca-stearat, 0,15 Teilen Zn-Stearat, 0,55 Teilen Diisodecyl-phenylphosphit und 0,3 Teilen des in Tabelle 3 angegebenen Stabilisators wird auf einem Mischwalzwerk 5 Minuten bei 180°C gewalzt. Vom gebildeten 0,3 mm dicken Walzfell werden Folienmuster in einem Testofen (®Mathis-Thermotester) bei 180°C thermisch belastet. Im angegebenen Zeitintervall wird an einem Prüfmuster der "Yellowness Index" (YI) nach ASTM D 1925 bestimmt. Die Ergebnisse sind in Tabelle 3 aufgeführt.

Tabelle 3

| Stabilisator | YI nach Belastungszeit in Minuten | | | |
|---|---|---|---|---|
| | 0 | 5 | 10 | 15 |
| Verbindung aus Beispiel 8 | 2,8 | 3,5 | 5,3 | 9,7 |
| Verbindung aus Beispiel 9 | 3,5 | 3,2 | 3,9 | 7,1 |

Beispiel 12: Eine Trockenmischung bestehend aus 100 Teilen S-PVC (®Vinnol H 70 DF), 17 Teilen Dioctylphthalat, 3 Teilen epoxidiertem Sojabohnenöl, 0,33 Teilen Zn-Oleat, 0,53 Teilen Ba-p-(t-butyl)-benzoat, 0,7 Teilen Diisodecyl-phenylphosphit, 0,44 Teilen ®SHELL SOL A (aromatisches Kohlenwasserstoff-Gemisch) und 0,2 Teilen des in Tabellen 4a und b angegebenen Stabilisators wird auf einem Mischwalzwerk 5 Minuten bei 190°C gewalzt. Vom gebildeten 0,3 mm dicken Walzfell werden

Folienmuster in Analogie an der in Beispiel 11 angegebenen Prüfmethode getestet. Die Ergebnisse sind in den Tabellen 4a und b zusammengefasst.

Tabelle 4a:

| Stabilisator | YI nach Belastungszeit in Minuten | | | | | | | | | | | | |
|---|---|---|---|---|---|---|---|---|---|---|---|---|---|
| | 0 | 5 | 10 | 15 | 20 | 25 | 30 | 35 | 40 | 45 | 50 | 55 | 60 |
| ohne | 8,4 | 11,9 | 14,4 | 18,4 | 22,0 | 23,2 | 22,8 | 20,5 | 19,1 | 19,0 | 21,8 | 27,8 | 36,7 |
| Verbindung aus Beispiel 1 | 0,6 | 1,3 | 1,7 | 3,0 | 6,5 | 9,6 | 12,0 | 13,3 | 13,8 | 14,8 | 15,8 | 18,4 | 25,2 |
| Verbindung aus Beispiel 2 | 1,1 | 1,4 | 2,5 | 4,6 | 7,5 | 10,5 | 12,0 | 12,7 | 13,4 | 14,4 | 16,1 | 19,7 | 26,1 |
| Verbindung aus Beispiel 3 | 0,9 | 1,4 | 1,9 | 3,4 | 6,7 | 9,6 | 11,3 | 12,2 | 12,8 | 13,4 | 16,6 | 17,3 | 23,2 |

Tabelle 4b:

| Stabilisator | YI nach Belastungszeit in Minuten | | | | | | | | | | | | |
|---|---|---|---|---|---|---|---|---|---|---|---|---|---|
| | 0 | 5 | 10 | 15 | 20 | 25 | 30 | 35 | 40 | 45 | 50 | 55 | 60 |
| Verbindung aus Beispiel 8 | 2,1 | 2,4 | 3,4 | 5,5 | 7,8 | 10,1 | 11,6 | 12,4 | 13,3 | 14,1 | 15,5 | 17,8 | 22,4 |
| Verbindung aus Beispiel 9 | 3,4 | 2,9 | 3,5 | 4,3 | 8,1 | 12,0 | 14,6 | 15,6 | 16,0 | 16,7 | 17,3 | 19,6 | 23,9 |

## Patentansprüche

1. Zusammensetzung enthaltend a) ein chlorhaltiges Polymerisat, b) mindestens ein Me(II)-Carboxylat und/oder Me(II)-Phenolat, wobei Me(II) Ba, Ca, Mg, Cd, Sr oder Zn bedeutet und c) mindestens eine Verbindung der Formel I,

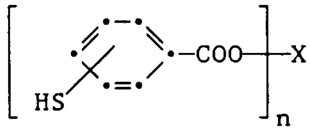 (I)

worin n 1, 2, 3, 4 oder 6 bedeutet und wenn n 1 ist, X $C_1$-$C_{20}$-Alkyl, $C_3$-$C_{20}$-Alkenyl, $C_5$-$C_{12}$-Cycloalkyl, durch $C_1$-$C_4$-Alkyl substituiertes $C_5$-$C_{12}$-Cycloalkyl, Phenyl, $C_7$-$C_9$-Phenylalkyl, durch 1 bis 3 Reste substituiertes Phenyl oder am Phenylring durch 1 bis 3 Reste substituiertes $C_7$-$C_9$-Phenylalkyl bedeutet, wobei die Reste aus der Gruppe bestehend aus $C_1$-$C_4$-Alkyl, Chlor, Hydroxy, Methoxy und Ethoxy ausgewählt sind; wenn n 2 ist, X $C_2$-$C_{12}$-Alkylen, durch Phenyl substituiertes $C_2$-$C_{12}$-Alkylen oder durch Sauerstoff- oder Schwefelatome unterbrochenes $C_2$-$C_{12}$-Alkylen bedeutet; wenn n 3 ist, X die Gruppe der Formel II

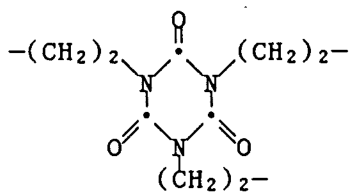 (II)

oder $C_3$-$C_7$-Alkantriyl darstellt; wenn n 4 ist, X $C_4$-$C_{10}$-Alkantetrayl oder die Gruppe der Formel IIIa oder IIIb ist

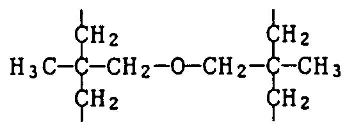 (IIIa)

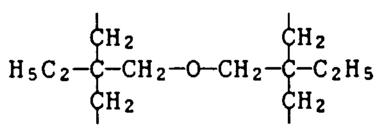 (IIIb)

und wenn n 6 bedeutet, X die Gruppe der Formel IV

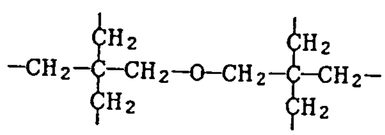 (IV)

darstellt,
mit den Bedingungen, dass

(1) Thiosalicylsäure-3-(3-t-butyl-4-hydroxyphenyl)-propylester als Komponente c) ausgeschlossen ist und

(2) die Zusammensetzung kein olefinisch ungesättigtes Terpen oder Sauerstoff-, Kohlenwasserstoff- oder Halogen-Derivat eines olefinisch ungesättigten Terpens enthält.

**2.** Zusammensetzung gemäss Anspruch 1, worin n 1, 2, 3 oder 4 bedeutet und wenn n 1 ist, X $C_1$-$C_{12}$-Alkyl, $C_3$-$C_{18}$-Alkenyl, $C_5$-$C_7$-Cycloalkyl, durch Methyl substituiertes Cyclohexyl, Phenyl oder $C_7$-$C_9$-Phenylalkyl bedeutet; wenn n 2 ist, X $C_2$-$C_6$-Alkylen darstellt; wenn n 3 ist, X die Gruppe der Formel II oder die Gruppe der Formel Va oder Vb

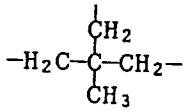 (Va) , 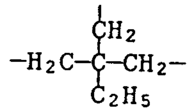 (Vb)

bedeutet und wenn n 4 ist, X die Gruppe der Formel Vc

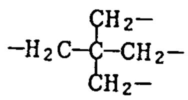 (Vc)

ist.

**3.** Zusammensetzung gemäss Anspruch 1 oder 2, worin n 1, 2, 3 oder 4 bedeutet und wenn n 1 ist, X $C_1$-$C_{12}$-Alkyl, Allyl, Methallyl, Oleyl, Cyclohexyl, Phenyl oder Benzyl bedeutet; wenn n 2 ist, X $C_2$-$C_6$-Alkylen darstellt; wenn n 3 ist, X die Gruppe der Formel Va oder Vb darstellt und wenn n 4 ist, X die Gruppe der Formel Vc bedeutet.

**4.** Zusammensetzung gemäss Anspruch 1, worin n 1 ist und X $C_1$-$C_{12}$-Alkyl bedeutet.

**5.** Zusammensetzung gemäss Anspruch 1, worin n 1 ist.

**6.** Zusammensetzung gemäss Anspruch 1, worin die Verbindung der Formel I 2-Ethylhexyl-thiosalicylat oder 1,6-Hexamethylen-bis[thiosalicylat] ist.

**7.** Zusammensetzung gemäss Anspruch 1, enthaltend als Komponente b) mindestens ein Me(II)-Carboxylat, wobei Me(II) Ba, Ca, Mg, Cd oder Zn bedeutet.

**8.** Zusammensetzung gemäss Anspruch 1, enthaltend als Komponente b) ein Gemisch aus Ba/Zn- oder Ca/Zn-Carboxylaten.

**9.** Zusammensetzung gemäss Anspruch 1, enthaltend als zusätzliche Komponente d) eine Epoxyverbindung und/oder ein Phosphit.

**10.** Zusammensetzung gemäss Anspruch 1, worin das chlorhaltige Polymerisat Polyvinylchlorid ist.

**11.** Verwendung von Verbindungen der Formel I gemäss Anspruch 1 zum Stabilisieren von chlorhaltigen Polymerisaten, welche mindestens ein Me(II)-Carboxylat und/oder Me(II)-Phenolat mit Me(II) = Ba, Ca, Mg, Cd, Sr oder Zn enthalten, gegen thermischen Abbau,
mit den Bedingungen, dass
(1) Thiosalicylsäure-3-(3-t-butyl-4-hydroxyphenyl)-propylester von den Verbindungen der Formel (I) ausgeschlossen ist und
(2) die chlorhaltigen Polymerisate kein olefinisch ungesättigtes Terpen oder Sauerstoff-, Kohlenwasserstoff- oder Halogen-Derivat eines olefinisch ungesättigten Terpens enthalten.

**12.** Verbindungen der Formel IA,

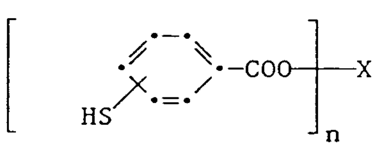 (IA)

worin n 2, 3, 4 oder 6 bedeutet und wenn n 2 ist, X durch Phenyl substituiertes $C_2$-$C_{12}$-Alkylen oder

durch Schwefelatome unterbrochenes $C_2$-$C_{12}$-Alkylen bedeutet; wenn n 3 ist, X die Gruppe der Formel II

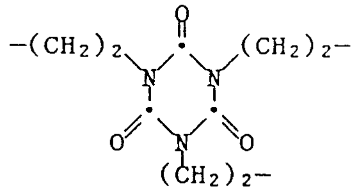

(II)

oder $C_3$-$C_7$-Alkantriyl darstellt; wenn n 4 ist, X $C_4$-$C_{10}$-Alkantetrayl oder die Gruppe der Formel IIIa oder IIIb ist

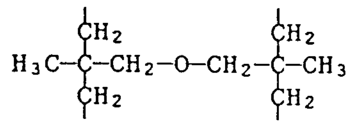

(IIIa)

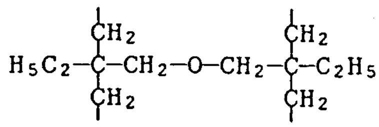

(IIIb)

und wenn n 6 bedeutet, X die Gruppe der Formel IV

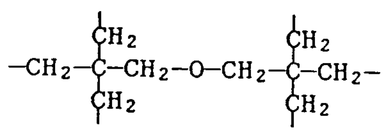

(IV)

darstellt.

**13.** Verbindungen gemäss Anspruch 12, worin n 3, 4 oder 6 bedeutet.

**Claims**

**1.** A composition comprising a) a chlorine-containing polymer, b) at least one Me(II) carboxylate and/or Me(II) phenolate, wherein Me(II) represents Ba, Ca, Mg, Cd, Sr or Zn, and c) at least one compound of formula I

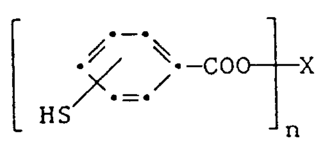

(I)

wherein n is 1, 2, 3, 4 or 6 and, when n is 1, X is $C_1$-$C_{20}$alkyl, $C_3$-$C_{20}$alkenyl, $C_5$-$C_{12}$cycloalkyl, $C_5$-$C_{12}$cycloalkyl substituted by $C_1$-$C_4$alkyl, phenyl, $C_7$-$C_9$phenylalkyl, phenyl substituted by from 1 to 3 radicals or $C_7$-$C_9$phenylalkyl substituted on the phenyl ring by from 1 to 3 radicals, the radicals being selected from the group consisting of $C_1$-$C_4$alkyl, chlorine, hydroxy, methoxy and ethoxy; when n is 2, X is $C_2$-$C_{12}$alkylene, $C_2$-$C_{12}$alkylene substituted by phenyl or $C_2$-$C_{12}$alkylene interrupted by oxygen or sulfur atoms; when n is 3, X is a group of formula II

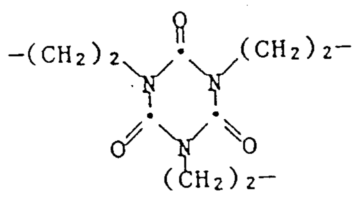

(II)

or is $C_3$-$C_7$alkanetriyl; when n is 4, X is $C_4$-$C_{10}$alkanetetrayl or a group of formula IIIa or IIIb

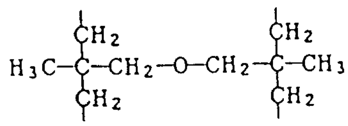

(IIIa)

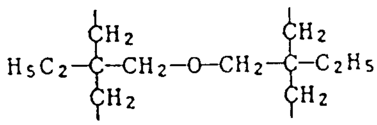

(IIIb)

and, when n is 6, X is a group of formula IV

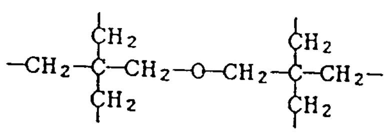

(IV),

with the restrictions that

(1) thiosalicyclic acid 3-(3-tert-butyl-4-hydroxyphenyl)-propyl ester is excluded as component c) and
(2) the composition does not contain an olefinically unsaturated terpene or an oxygen, hydrocarbon or halogen derivative of an olefinically unsaturated terpene.

**2.** A composition according to claim 1, wherein n is 1, 2, 3 or 4 and, when n is 1, X is $C_1$-$C_{12}$alkyl, $C_3$-$C_{18}$alkenyl, $C_5$-$C_7$cycloalkyl, methyl-substituted cyclohexyl, phenyl or $C_7$-$C_9$phenylalkyl; when n is 2, X is $C_2$-$C_6$alkylene; when n is 3, X is a group of formula II or a group of formula Va or Vb

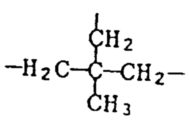

(Va) ,

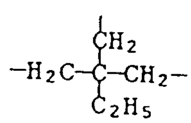

(Vb)

and, when n is 4, X is a group of formula Vc

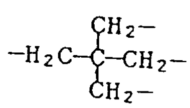

(Vc).

**3.** A composition according to claim 1 or claim 2, wherein n is 1, 2, 3 or 4 and, when n is 1, X is $C_1$-$C_{12}$alkyl, allyl, methallyl, oleyl, cyclohexyl, phenyl or benzyl; when n is 2, X is $C_2$-$C_6$alkylene; when n is 3, X is a group of formula Va or Vb and, when n is 4, X is a group of formula Vc.

**4.** A composition according to claim 1, wherein n is 1 and X is $C_1$-$C_{12}$alkyl.

**5.** A composition according to claim 1, wherein n is 1.

**6.** A composition according to claim 1, wherein the compound of formula I is 2-ethylhexyl thiosalicylate or 1,6-hexamethylene bis[thiosalicylate].

**7.** A composition according to claim 1, comprising as component b) at least one Me(II) carboxylate, wherein Me(II) represents Ba, Ca, Mg, Cd or Zn.

**8.** A composition according to claim 1, comprising as component b) a mixture of Ba/Zn carboxylates or Ca/Zn carboxylates.

**9.** A composition according to claim 1, comprising as an additional component d) an epoxy compound and/or a phosphite.

**10.** A composition according to claim 1, wherein the chlorine-containing polymer is polyvinyl chloride.

**11.** The use of a compound of formula I according to claim 1 for stabilising chlorine-containing polymers that incorporate at least one Me(II) carboxylate and/or Me(II) phenolate, wherein Me(II) represents Ba, Ca, Mg, Cd, Sr or Zn, against thermal degradation,
with the restrictions that
(1) thiosalicylic acid 3-(3-tert-butyl-4-hydroxyphenyl)-propyl ester is excluded from the compounds of formula (I) and
(2) the chlorine-containing polymer does not contain an olefinically unsaturated terpene or an oxygen, hydrocarbon or halogen derivative of an olefinically unsaturated terpene.

**12.** A compound of formula IA

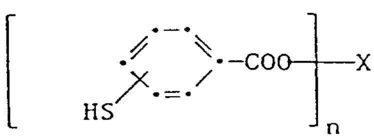

(IA)

wherein n is 2, 3, 4 or 6 and, when n is 2, X is $C_2$-$C_{12}$-alkylene substituted by phenyl or is $C_2$-$C_{12}$alkylene interrupted by sulfur atoms; when n is 3, X is a group of formula II

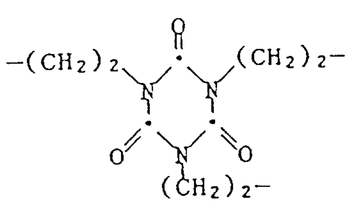

(II)

or $C_3$-$C_7$alkanetriyl; when n is 4, X is $C_4$-$C_{10}$alkanetetrayl or a group of formula IIIa or IIIb

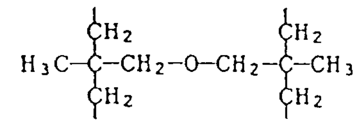

(IIIa)

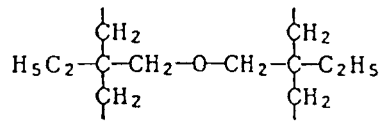

(IIIb)

and, when n is 6, X is a group of formula IV

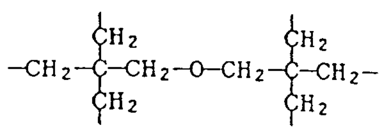

(IV).

**13.** A compound according to claim 12, wherein n is 3, 4 or 6.

**Revendications**

**1.** Composition comportant

a) un polymère chloré,

b) au moins un carboxylate de Me(II) et/ou phénolate de Me(II), où Me(II) représente Ba, Ca, Mg, Cd, Sr ou Zn, et

c) au moins un composé de formule I,

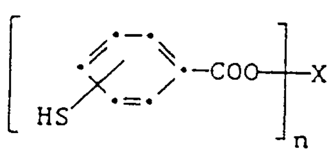

(I)

où n représente 1, 2, 3, 4 ou 6, et

si n est 1, X représente un alkyle en $C_1$-$C_{20}$, un alcényle en $C_3$-$C_{20}$, un cycloalkyle en $C_5$-$C_{12}$, un cycloalkyle en $C_5$-$C_{12}$substitué par un alkyle en $C_1$-$C_4$, le phényle, un phénylalkyle en $C_7$-$C_9$, un phényle substitué par de 1 à 3 restes, ou un phénylalkyle en $C_7$-$C_9$ substitué sur le cycle du phényle par de 1 à 3 restes, les restes étant choisis dans le groupe constitué par un alkyle en $C_1$-$C_4$, le chlore, l'hydroxy, le méthoxy et l'éthoxy ;

si n est 2, X représente un alkylène en $C_2$-$C_{12}$, un alkylène en $C_2$-$C_{12}$ substitué par le phényle ou un alkylène en $C_2$-$C_{12}$ interrompu par un atome d'oxygène ou de soufre ;

si n est 3, X représente le groupe de formule II

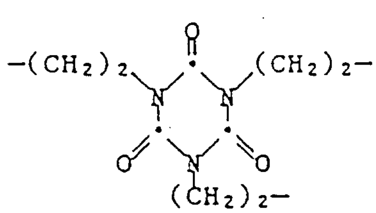

(II)

ou un alcane triyle en $C_3$-$C_7$ ;

si n est 4, X représente un alcane tétrayle en $C_4$-$C_{10}$ ou le groupe de formule IIIa ou IIIb

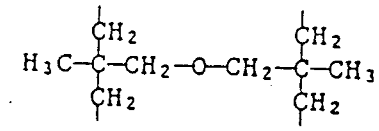 (IIIa)

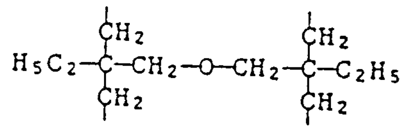 (IIIb)

et si n est 6, X représente le groupe de formule IV

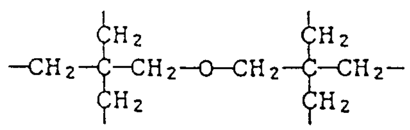 (IV)

avec les conditions suivantes :

(1) l'ester 3-(3-t-butyl-4-hydroxyphényl)-propylique de l'acide thiosalicylique est exclu comme composant c), et

(2) la composition ne contient aucun terpène à insaturation oléfinique ou aucun dérivé avec l'oxygène, un hydrocarbure ou un halogène d'un terpène à insaturation oléfinique.

**2.** Composition selon la revendication 1, où n est 1, 2, 3 ou 4 et
si n est 1, X représente un alkyle en $C_1$-$C_{12}$, un alcényle en $C_3$-$C_{18}$, un cycloalkyle en $C_5$-$C_7$, un cyclohexyle substitué par le méthyle, le phényle ou un phénylalkyle en $C_7$-$C_9$ ;
si n est 2, X représente un alkylène en $C_2$-$C_6$ ;
si n est 3, X représente le groupe de formule II ou le groupe de formule Va ou Vb

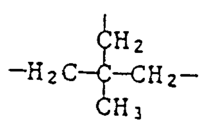 (Va) , 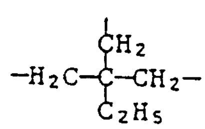 (Vb)

et si n est 4, X représente le groupe de formule Vc

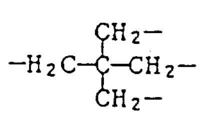 (Vc)

**3.** Composition selon la revendication 1 ou 2, où n est 1, 2, 3 ou 4 et
si n est 1, X représente un alkyle en $C_1$-$C_{12}$, l'allyle, le méthallyle, l'oléyle, le cyclohexyle, le phényle ou le benzyle ;
si n est 2, X représente un alkylène en $C_2$-$C_6$ ;
si n est 3, X représente le groupe de formule Va ou Vb, et
si n est 4, X représente le groupe de formule Vc.

**4.** Composition selon la revendication 1, où n est 1 et X représente un alkyle en $C_1$-$C_{12}$.

**5.** Composition selon la revendication 1, où n est 1.

**6.** Composition selon la revendication 1, où le composé de formule I est le thiosalicylate de 2-éthylhexyle ou le 1,6-hexaméthylène-bis[thiosalicylate].

**7.** Composition selon la revendication 1, comportant comme composant b) au moins un carboxylate de Me(II), où Me(II) représente Ba, Ca, Mg, Cd ou Zn.

**8.** Composition selon la revendication 1, comportant comme composant b) un mélange de carboxylates de Ba/Zn ou de Ca/Zn.

**9.** Composition selon la revendication 1, comportant comme composant d) supplémentaire un composé époxy et/ou un phosphite.

**10.** Composition selon la revendication 1, où le polymère chloré est le polychlorure de vinyle.

**11.** Utilisation de composés de formule I selon la revendication 1, pour stabiliser des polymères chlorés, qui comportent au moins un carboxylate de Me(II) et/ou phénolate de Me(II) , avec Me(II) = Ba, Ca, Mg, Cd, Sr ou Zn, pour lutter contre la dégradation thermique, avec les conditions suivantes :
(1) l'ester 3-(3-t-butyl-4-hydroxyphényl)-propylique de l'acide thiosalicylique est exclu des composés de formule (I), et
(2) les polymères chlorés ne contiennent aucun terpène à insaturation oléfinique ou aucun dérivé avec l'oxygène, un hydrocarbure ou un halogène, d'un terpène à insaturation oléfinique.

**12.** Composés de formule IA

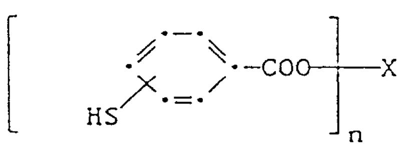

(IA)

où n est 2, 3, 4 ou 6 et
si n est 2, X représente un alkylène en $C_2$-$C_{12}$ substitué par le phényle ou un alkylène en $C_2$-$C_{12}$ interrompu par un atome de soufre ;
si n est 3, X représente le groupe de formule II

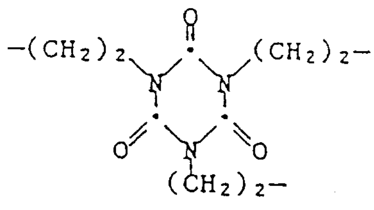

(II)

ou un alcane triyle en $C_3$-$C_7$ ;
si n est 4, X représente un alcane tétrayle en $C_4$-$C_{10}$ ou le groupe de formule IIIa ou IIIb.

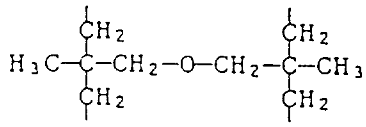 (IIIa)

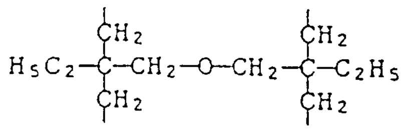 (IIIb)

et si n est 6, X représente le groupe de formule IV

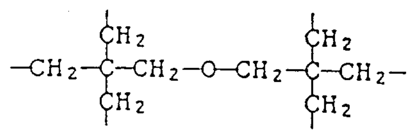 (IV)

**13.** Composés selon la revendication 12, où n est 3, 4 ou 6.